# EUROPÄISCHE PATENTSCHRIFT

(11) **EP 0 988 018 B1**
(45) Veröffentlichungstag und Bekanntmachung des Hinweises auf die Patenterteilung: **05.03.2003**
(21) Anmeldenummer: 99924664.8
(22) Anmeldetag: 22.03.1999
(51) Int. Cl.: A61K 6/00

(54) **HAFTMITTEL FÜR ZAHNPROTHESEN**
ADHESIVE FOR DENTAL PROSTHESES
AGENT ADHESIF POUR PROTHESES DENTAIRES

(30) Priorität: 07.04.1998 DE 19815547
(43) Veröffentlichungstag der Anmeldung: 29.03.2000
(73) Patentinhaber: Schmidt, Joachim, 77797 Ohlsbach (DE)
(72) Erfinder: SCHMIDT, Joachim, D-77797 Ohlsbach (DE); UNRUH, Martin, D-77960 Seelbach (DE); SCHMIDT, Torsten, St. Vincent und die Grenadinen (VC)
(74) Vertreter: Kern, Gerbert
(86) Internationale Anmeldenummer: DE9900824
(87) Internationale Veröffentlichungsnummer: WO99051189

(56) Entgegenhaltungen:
- DE-C- 728 003
- FR-A- 2 335 203
- FR-A- 2 486 392
- US-A- 3 410 704
- US-A- 5 275 834

## Beschreibung

Die Erfindung betrifft Haftmittel hauptsächlich aus Pektinen oder Pektinaten für Zahnprothesen, der im einleitenden Teil des Anspruchs 1 angegebenen Art.

In DE-A 2316603 wird eine Mischung unter Hinweis auf eine anteilmäßige Mitverwendung von A mit etwa 1 bis etwa 80 Gew.%, mindestens eines kationischen Polymeren, bestehend aus
(1) einem Acrylamid
(2) einem Mischpolymeren
aus einem Acrylamid, etc.....
und B etwa 20 bis etwa 99 Gew.%, mindestens eines anionischen Gummis wie: Karayagummi, Gummiarabicum, Shiraz-Gummi, Traganth, Pektin, Pektinat, Algin, einem Alginat, einem Carboxy methylcellulose-Gummi, einem Cellulosesulfat-Gummi oder einem synthetischen gummiartigen Mischpolymeren aus Maleinsäureanhydrid und einem Vinylalkyläther mit 1 bis 5 Kohlenstoffatomen, genannt.

Aus den Druckschriften DE - PS 728003 und US 3410704 sind Haftmittel auf Pektinbasis bekannt.

Die in der Literatur vorgeschlagenen Haftmittel für Zahnprothesen weisen neben ersten Formulierungen auf Basis von Exsudaten und deren Gemischen heute vornehmlich synthetische oder chemisch modifizierte Produkte als Basisstoffe auf.

Zu nennen sind hier.
Cellulose-Derivate wie Natriumcarboxymethylcellulosen, Hydroxyalkylcellulosen, Polyvinylalkohol, Polyvinylacetat, Polyvinyläther, Polyvinylpyrrolidon, Polyacrylamide, Polymethacrylsäure-Derivate, Copolymerisate aus Maleinsäureanhydrid und Vinyläthern und deren Salzen; chemisch modifizierte Alginsäuren wie die Natrium,-Kalium,-Ammoniumsalze (Alginate); chemisch modifizierte Stärken wie: Quellstärke, kaltwasserlösliche oder teilwasserlösliche Stärke, oxidierte Stärke, sowie Stärkehydrolysate.

Die aufgeführten Produkte finden in Haftmitteln wie Haftpulvern, Haftcremes, Haftlösungen etc. als alleiniger Basisstoff oder aber in Abmischungen, Anwendung. Doch haften all diesen Mitteln und den daraus gefertigten Haftmitteln Nachteile an.

Besonders hervorzuheben sind:
- A: Ungenügende Anfangshaftfestigkeit nach Einsetzen der mit Haftmittel versehenen Zahnprothese unter kurzem, kräftigem Andruck und ca. 10 Sec. Festhaltedauer im Gaumen-Rachen-bereich. Eine Nichteinhaltung der Mindestwartezeit von ca. 30 bis 60 Minuten durch vorzeitiges Zähneputzen, Essen und/oder Getränkeaufnahme, hat gegebenenfalls eine Loslösung der Prothese und das erneute Einsetzen der Prothese, unter erneutem Einsatz von Haftmittelsubstanz, zur Folge.
- B: Bei Entnahme der Zahnprothese verbleiben am Gaumen, auch dem Prothesekörper, stark klebende oder unangenehm schleimende oder schwer zu entfernende Haftmittel-Rückstände
und/oder
- C: geschmackliche, insbesondere unter seifigem Geschmack sich lösende/ablösende Haftmittelanteile.
- D: Ungenügende Reserve an Haftfestigkeitspotential des Haftmittels mit der Folge einer ev. Nachbesserung mit Haftmittelsubstanz, ggfls. bedingt durch ungenügendenden Haftmittelauftrag (Unterdosierung) besonders gegeben bei einem älteren Personenkreis.

Aufgabe der vorliegenden Erfindung ist es, die unter A bis D genannten Nachteile bei Haftmitteln für Zahnprothesen zu überwinden. Es wurde nun gefunden, daß spezielle Pektine in hohem Maße zur Lösung der erfindungsgemäßen Aufgabe geeignet sind.

Die Lösung der Aufgabe besteht darin, daß das Haftmittel ein nicht gelierendes, dominant Acetylgruppen enthaltendes Rübenpektin mit einem Molekulargewicht von 49000 bis 90000 Dalton und/oder ein niederverestertes, Säureamidgruppen enthaltendes und calciumreaktiv gelierendes Pektin oder Pektinat aufweist, wie im beigefügten Anspruch 1 angegegeben.

Pektin ist ein Naturprodukt. Es ist ein Strukturelemet aller höheren Landpflanzen. In der Mittellamelle einer Schicht, die zwischen den Zellen die Zellwände von Obst und Gemüse zusammenhält, ist der Pektingehalt am höchsten. Es übernimmt in der Struktur als Kittsusbtanz wichtige Stütz -und Festigungsfunktionen und regelt durch seine starke Quellfähigkeit und Kolloidnatur den Wasserhaushalt der Früchte.

Chemisch betrachtet ist Pektin eine makromolekulare Verbindung, die zu den Heteropolysacchariden zählt. Die Pektinmoleküle sind unverzweigte, lineare Ketten aus im Mittel 200-1000 Galakturonsäure-Einheiten, die Alpha-1,4 glykosidisch miteinander verbunden sind. Hauptbestandteil ist Polygalakturonsäure, die anteilsmäßig mit Methanol zu Polygalakturonsäuremethylester verestert, vorkommt (Veresterungsgrad).

Der Veresterungsgrad DE als charakteristische Größe des Pektins ist definiert als das Verhältnis der Menge der mit Methanol veresterten Galakturonsäure-Einheiten zur gesamten Anzahl der Galakturonsäure-Einheiten im Molekül.

Pektin mit einem Veresterungsgrad von DE 50% und darüber wird als HV-hochverestertes Pektin oder HM-High Methyl-Pektin eingestuft.

Der DE Veresterungsgrad von kommerziell hochverestertem Pektin liegt zwischen 58 % und 75 %.
Chemische Struktur von hochverestertem Pektin, DE 75%:

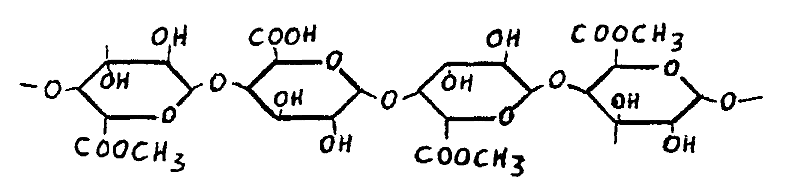

Pektin mit einem Veresterungsgrad unter DE 50% wird als NV niederverestertes Pektin oder LM Low Methyl-Pektin bezeichnet. Die NV niederveresterten Pektine lassen sich aufteilen in zwei Untergruppen:
- die konventionellen niederveresterten LMC-Pektine
   und die
- amidierten niederversterten LMA-Pektine.

Chemische Struktur von niederverestertem Pektin, DE 25 %: 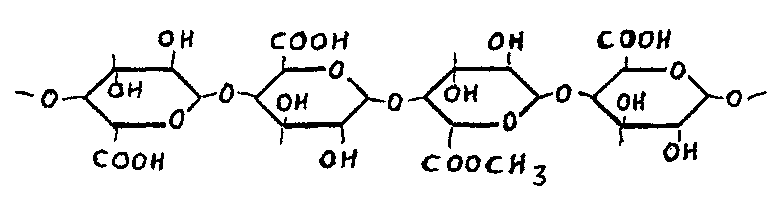 Chemische Struktur von amidiertem, niederverestertem Pektin,
DA 25 %, DE 25 %:

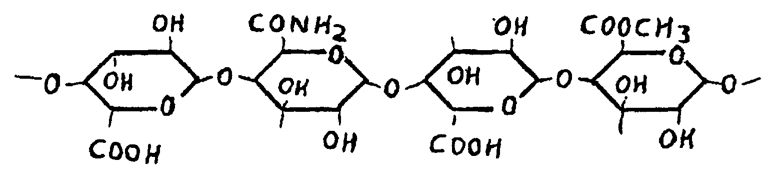

Ein unterscheidendes Merkmal und Charakteristikum der LMC, insbesondere der LMA niederveresterten Pektine, zu den konventionell HM-hochveresterten Pektinen ist ihre ausgeprägte Empfindlichkeit und Reaktivität mit zwei-und mehrwertigen Kationen wie beispielsweise mit zweiwertigen Calcium,-Eisen-Zinkionen oder dreiwertigen Eisen,-Aluminiumionen etc. LMC-Pektine sind entscheidend weniger Calciumreaktiv als LMA-Pektine und benötigen die drei-bis vierfache Menge an Calciumionen für den Aufbau einer Gelstruktur. Bei geringem Calciumanteil stehen bei den LMC-Pektinen nur rein verdickende Eigenschaften im Vordergrund. Die Herstellung der NV-niederveresterten Pektine erfolgt durch Teilentesterung, immer ausgehend von einem HVhochveresterten Pektin, in mild saurem Medium zu konventionell LMC-Pektinen und in ammoniakalischem Milieu zu LMA-Pektinen.

Da jede Entesterungsprozedur mit einem Teilabbau des Polymerisationsgrades bzw. einem Molekulargewichtsabbau einhergeht, ist eine zu weit geführte Teilentesterung für das erfindungsgemäße Haftmittel nicht sinnvoll. Mit zunehmender Entesterung, aufgrund freigesetzter Carboxylgruppenhaltiger Galakturonsäure-Einheiten, nimmt die Kationenreaktivität zu. Überreaktionen und ein Übermaß an Pektinatausfällungen können die Folge sein. Es wurde nun überraschend festgestellt, daß eine hohe Kationensensibilität, vorzugsweise Calciumreaktivität bei einem relativ hohen Molekulargewicht das Haftmittel verfestigende Wirkungen z. Bsp. in Gegenwart des Speichels auslöst. Bereits bei geringer Calciumionenzugabe beginnen sich die Pektinmolekülketten über Calciumbrücken zusammenzulagern; bei weiterer Calciumionenkonzentration treten Geliervorgänge ein und bei Überdosierung fällt unter den gegebenen Gelierbedingungen gegebenenfalls Calciumpektinat aus.

Eine ausgeprägte Calciumsensibilität und ein relativ hohes Molekulargewicht aufweisende LMA niederveresterte und amidierte Pektine, lassen sich aus einer Vielzahl der heute durch moderne Forschung auf dem Markt befindlichen derartigen Pektine herausfiltern. Die Calciumreaktivität nimmt mit steigendem DA Amidierungsgrad und abnehmendem DE Veresterungsgrad zu. Die Calciumreaktivät wird unterteilt in Hoch, Mittel und Niedrig sowohl für LMA als auch LMC Pektine.

| DE, % | DA, % | Calciumreaktivität |
|---|---|---|
| 36 | 14 | niedrig |
| 33 | 17 | mittel |
| 30 | 20 | hoch |
| 48 | - | niedrig |
| 40 | - | mittel |
| 34 | - | hoch |

Für die erfindungesgemäß formulierten Haftmittel ist in diesem Zusammenhang von Bedeutung, daß der Speichel neben Anionen wie Chloriden, Phosphaten und Bicarbonaten; Kationen wie Calcium, Natrium, Kalium etc. enthält, die wie ausgeführt wirken können. Pektine werden aus natürlicher pflanzlicher Rohware mit hohem Pektingehalt vornehmlich aus Apfel- und Citrus-Pressrückständen oder aber in untergeordneten Mengen aus den ausgedroschenen Fruchtständen von Sonnenblumen oder entzuckertem Rübentrester bei der Zuckerfabrikation durch mild sauer oder alkalisch eingestellte wässrige Extraktion gewonnen. Je nach Qualität der Rohware besitzt das Pektin unterschiedliche Eigenschaften. Die abschließende Isoliering des Pektins wird durch eine Fällung in Alkohol erreicht. Das ausgefällte Pektin wird anschließend getrocknet, gemahlen und gegebenenfalls standardisiert. Alle Pektine, die für die Verwendung in der Konfitürenindustrie vorgesehen sind, werden hinsichtlich ihrer Gelstärke und Geliergeschwindigkeit-- Auslieferung einer gleichbleibenden Qualität an den Kunden - standardisiert. Dabei werden verschiedene Chargen, die sich in ihren Geliereigenschaften leicht unterscheiden können, untereinander gemischt und anschließend mit vornehmlich Einfach-oder Mehrfachzuckern, insbesondere mit Dextrose oder Saccharose, auf die gewünschte Geltsärke eingestellt. Der Zuckeranteil bewegt sich in der Regel zwischen
25 bis 35 %.

Mit Hilfe modernster, wissenschaftlicher Methoden ist es besonders in jüngster Zeit gelungen, die Strukturen der Pektinsorten immer genauer zu analysieren, so daß heute unbegrenzte Möglichkeitem zur Entwicklung von Spezialpektinen für alle noch so spezifischen Anwendungsbereiche bestehen.

Die Unbedenklichkeit der Pektine für den Menschen, seinen Verzehr, steht außer Frage. Hervorzuheben sind die blutstillenden und hellungsfördernden Wirkungen alleine oder in Kompositionen (Phlogistik). Pektine sind zudem in der Lage, den Anstieg des Serum-, Glukosespiegels zu verlangsamen und den Cholesterinspiegel zu senken.

Überraschenderweise wurde nun gefunden, daß durch einen schonenden Teilentesterungsprozess in ammoniakalisch wässriger Phase sich Poly-Galakturonsäureamide ausbilden, so daß die Pektinmolekülkette hier außer Poly-Galakturonsäure und Poly-Galakturonsäuremethylester zusätzlich Poly-Galakturonsäureamide enthält, sich außerordentlich verbessernd auf die Haftfähigkeit/Haftfestigkeit im Haftmittel auswirkt.

Weiters wurde gefunden, daß die Höhe des Molekulargewichts bzw. des Polymerisationsgrades der Pektin-Makromolekülkette hierbei von Bedeutung ist. Ein relativ hohes Molekulargewicht wirkt unterstützend und Haftkraft steigernd auf die Ausbildung des Pektinnervs und damit auf die Haftfestigkeit und seine Zeitdauer im Haftmittel auf Prothese und Gaumen im Rachenraum.

Anhand durchgeführter Versuche an Probanden, die Oberkieferprothesen tragen, wurde die Überlegenheit der erfindungsgemäßen Haftmittel, der unter A bis D aufgeführten Negativkriterien nachgwiesen.

### VERGLEICHSVERSUCHE betreffend die ANFANGSHAFTFESTIGKEIT und das HAFTFESTIGKEITSPOTENTIAL

Auf die gereinigte und noch feuchte Zahnprothese wurde Haftpulver oder Haftcreme nach Gebrauchsanweisung aufgetragen. Die Prothese wurde mit kurzem, kräftigem Andruck und 10 Sec. Haltedauer im Oberkiefer-Gaumenbereich eingesetzt. Die Prüfung begann um 10 Uhr morgens. Beurteilt wurde die Anfangshaftfestigkeit sofort nach Einsatz der Prothese (10 Sec.), sowie alle danach folgenden 10 Min. bis zu einer Gesamtzeitdauer von 1 Stunde. Nach 1 Stunde zeigten alle mit Haftpulver eingesetzten Prothesen relativ festen Halt. Haftcremes hingegen ließen auch weiterhin eine leichte Verschiebung zu.

Unter Auflegung des Zeigefingers im oberen Teil des Nasenrückens zur Finger-Haltungsfixierung, wurde mit dem Daumen hinter den Zähnen ansetzend und Druck ausübend versucht, die Oberkieferprothese nach vorne zu schieben, bzw. nach unten in den Rachenraum zu drücken.

Die Ausführung und Einsetzung der Oberkieferprothese erfolgte in gleicher Weise, wie bei der Anfangshaftfestigkeitsprüfung beschrieben. Der Nacht-Tageszyklustest begann um 21 Uhr abends. Die erste Prüfung erfolgte nach 12 Stunden am nächsten Morgen um 9 Uhr. Geprüft wurde danach alle weiteren 2 Stunden bis zu einer Gesamtzeitdauer von 24 Stunden um 21 Uhr abends des darauffolgenden Tages. Haftmittel, deren Haftpotential darüber hinaus lag, wurden im gleichen Ablauf nach dem Tages-Nachtzyklus geprüft mit geänderter Anfangszeit. Beginn 9 Uhr morgens.

Die Ausführung des Fingertests erfolgte wie bereits ausgeführt unter Anfangshaftfestigkeit. Eine deutlich spürbare und bleibende Lockerung bzw. Schubbeweglichkeit der Prothese beendeten den Versuch.

Dem Test wurden marktgängige Haftpulver und Haftcremes unterzogen. Es ließ sich eine Einteilung treffen in:

| | | |
|---|---|---|
| I) | Haftpulver auf Basis | Alginaten (Natrium, Kalium, Ammoniumsalze) |
| II) | " | Natrium - Carboxymethylcellulosen = Na-CMC, mittel- und/oder hochviskos |
| III) | " | Alginatanteil und/oder Natrium-Carboxymethylcelluloseanteil und Gelatineanteil |
| IV) | Haftcremes auf Basis | Salze von Alkylvinylether/Maleinsäureanhydrid-Copolymeren (AVE/MSA) |

### BEISPIELE

### HAFTPULVER

| Beispiel 1 | |
|---|---|
| Rüben-Reinpektin | 100g |
| DE-Veresterungsgrad | 55% |
| Acetylgruppenanteil | 21% |
| Molekulargewicht | 65000 Dalton |
| Nicht gelierend | |

| Beispiel 2 | |
|---|---|
| Citrus-Reinpektin amidiert | 100 g |
| DE Veresterungsgrad | 30% |
| DA Amidierungsgrad | 20% |
| Molekulargewicht | 90000 Dalton |
| Calciumreaktivität | hoch |

| Beispiel 3 | |
|---|---|
| Citrus-Pektin amidiert | 100 g |
| Standardisiert | (30% Saccharose) |
| DE Veresterungsgrad | 30% |
| DA Amidierungsgrad: | 20% |
| Molekulargewicht: | 90000 Dalton |
| Calciumreaktivität | hoch |

Anstelle von Saccharose können gewählt werden Dextrose oder andere Zucker-oder Stärkehydrolysate.

### HAFTCREME

| BEISPIEL 4 | |
|---|---|
| Rüben-Reinpektin (nach Bsp. 1) | 50,0g |
| Pharm. Vaseline | 30,0g |
| Pharm. Paraffinöl | 20,0g |
| Kaliumsorbat 1 Promille | 0,1g |

| Beispiel 5 | |
|---|---|
| Citrus-Reinpektin amidiert (nach Bsp.2) | 41,18g |
| Pharm. Vaseline | 35,29g |
| Pharm. Paraffinöl | 23,53 g |
| Kaliumsorbat | 0,10 g |

| AUSWERTUNG | | | | | |
|---|---|---|---|---|---|
| | Anfangshaftfestigkeit A | Haftmittelrückstände B | Haftmittel Geschmack C | Haftfestigkeit Potential D | Auftragsmenge (mg) E |
| I) Alginate | 40 - 60 min. | Keine | Rel. stark | 18 - 20 Std. | 500 |
| | | | seifig | 4 Std. | 250 |
| II) Na - CMC | 30 - 50 min. | Keine | seifig | 15 - 18 Std. | 500 |
| | | | | 14 Std. | 250 |
| III) Alginatant, und/oder Na-CMC-/u. Gelatineant. | 30 - 60 min. | Keine | seifig | 16 - 20 Std. | 500 |
| | | | | 13 - 14 Std. | 250 |
| IV) Haftcremes | 50 - 70 min. | Gaumen u. Prothese | Keine | 15 - 20 Std. | 1000 (40-50%) |

| Haftpulver: Nach der Erfindung | | | | | |
|---|---|---|---|---|---|
| | Anfangshaftfestigkeit A | Haftmittel-Rückstände B | Haftmittel Geschmack C | Haftfestigkeit Potential D | Auftragsmenge (mg) |
| Beispiel 1 | sofort | Keine | Keine | 32 Std. | 500 |
| | sofort | Keine | Keine | 24 Std. | 250 |
| | sofort | Keine | Keine | 12 Std. | 100 |
| | | | | | |
| Beispiel 2 | sofort | Keine | Keine | 48 Std. | 500 |
| | sofort | Keine | Keine | 34 Std. | 250 |
| | sofort | Keine | Keine | 24 Std. | 100 |
| | | | | | |
| Beispiel 3 | sofort | Keine | Keine | 31 Std. | 500 |

| Haftcreme: Nach der Erfindung | | | | | |
|---|---|---|---|---|---|
| Beispiel 4 | bis 30 Min. | Keine | Keine | 20 - 22 Std. | 1000 (40-50%) |
| | | | | | |
| Beispiel 5 | bis 30 Min. | Keine | Keine | 22 - 25 Std. | 1000 (40-50%) |

## Patentansprüche

1. Haftmittel, hauptsächlich aus Pektinen oder Pektinaten für Zahnprothesen **dadurch gekennzeichnet, daß** es ein nicht gelierendes, dominant Acetylgruppen enthaltendes, Rübenpektin mit einem Molekulargewicht nach Dalton von mindestens 49000 bis 90000 und/oder ein niederverestertes, Säureamidgruppen enthaltendes und calciumreaktiv gelierendes Pektin oder Pektinat aufweist.

2. Haftmittel nach Anspruch 1 **dadurch gekennzeichnet, daß** das Pektin oder Pektinat in seiner Molekülkette neben vorhandener Poly-Galakturonsäure-Einheiten und PolyGalakturonsäuremethylester-Einheiten Säureamidgruppen als Poly-Galakturonsäureamid-Einheiten besitzt, bei einem Molekulargewicht größer als 40 000 Dalton.

3. Haftmittel nach Anspruch 2 **dadurch gekennzeichnet, daß** der Amidierungsgrad im Bereich von 14 bis 35%, bevorzugt 20 bis 25% und das Molekulargewicht 70 000 bis 150 000 Dalton beträgt.

4. Haftmittel nach Anspruch 2 und 3 **dadurch gekennzeichnet, daß** das amidierte Pektin oder Pektinat hoch calciumreaktiv ist.

5. Haftmittel für Zahnprothesen nach den Ansprüchen 1 bis 4 **dadurch gekennzeichnet, daß** das Pektin oder Pektinat mit organischen und/oder anorganischen Stoffen, verschnitten ist.

## Claims

1. An adhesive, primarily comprising pectins or pectinates for dental prostheses, **characterised in that** it has a non-gelling beet pectin, containing dominant acetyl groups, having a Dalton molecular weight of at least 49000 to 90000 and/or a low-esterified pectin or pectinate which contains acid amide groups and gels in reaction with calcium.

2. The adhesive as claimed in Claim 1, **characterised in that** the pectin or pectinate in its molecular chain has, apart from available poly-galacturonic acid units and poly-galacturonic acid methylester units, acid amide groups as poly-galacturonic acid amide units, at a molecular weight greater than 40 000 Dalton.

3. The adhesive as claimed in Claim 2, **characterised in that** the degree of amidation is in the region of 14 to 35%, preferably 20 to 25% and the molecular weight is 70 000 to 150 000 Dalton.

4. The adhesive as claimed in Claim 2 and 3, **characterised in that** the amidised pectin or pectinate is highly reactive to calcium.

5. The adhesive for dental prostheses as claimed in Claims 1 to 4, **characterised in that** the pectin or pectinate is blended with organic and/or inorganic substances.

## Revendications

1. Agent adhésif, constitué principalement de pectines ou pectinates, pour prothèses dentaires, **caractérisé par le fait qu'**il présente une pectine de betteraves contenant un groupe acétyle dominant non gélifiant, dont le poids moléculaire selon Dalton est d'au moins 49 000 à 90 000 et/ou une pectine ou pectinate de basse estérification, contenant des groupes d'amides d'acides, gélifiant et réactif au calcium.

2. Agent adhésif selon la revendication 1, **caractérisé par le fait que** la pectine ou pectinate possède dans ses chaînes moléculaires, à côté des unités existantes d'acide polygalacturonique et d'ester de méthyle polygalacturonique sous forme d'unités d'amides d'acides polygalacturonique, présentant un poids moléculaire selon Dalton supérieur à 40 000.

3. Agent adhésif selon la revendication 2, **caractérisé par le fait que** le degré d'amidation se situe dans une plage entre 14 et 35 %, de préférence entre 20 et 25 % et le poids moléculaire entre 70 000 et 150 000 Dalton.

4. Agent adhésif selon les revendications 2 et 3, **caractérisé par le fait que** la pectine ou pectinate amidée est hautement réactive au calcium.

5. Agent adhésif pour prothèses dentaires selon les revendications 1 à 4, **caractérisé par le fait que** la pectine ou pectinate est coupée avec des substances organiques et/ou inorganiques.
